(19) Europäisches Patentamt
European Patent Office
Office européen des brevets

(11) Publication number: **0 180 266**
**B1**

(12) # EUROPEAN PATENT SPECIFICATION

(45) Date of publication of patent specification: **23.11.89**

(51) Int. Cl.$^4$: **C 07 C 43/11**, C 07 C 41/03

(21) Application number: **85201593.2**

(22) Date of filing: **02.10.85**

(54) **Alkoxylation process using bimetallic oxo catalyst.**

(30) Priority: **29.10.84 US 666062**

(43) Date of publication of application:
**07.05.86 Bulletin 86/19**

(45) Publication of the grant of the patent:
**23.11.89 Bulletin 89/47**

(84) Designated Contracting States:
**BE CH DE FR GB IT LI NL**

(56) References cited:
**EP-A-0 020 867**
**EP-A-0 082 554**
**EP-A-0 104 309**
**DE-A-2 639 564**
**US-A-3 432 445**
**US-A-3 607 785**
**US-A-4 210 764**
**US-A-4 281 087**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Edwards, Charles Lee**
**12526 Piping Rock**
**Houston Texas 77077 (US)**

(74) Representative: **Aalbers, Onno et al**
**P.O. Box 302**
**NL-2501 CH The Hague (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 180 266 B1

**Description**

This invention relates to a process for the preparation of alkylene oxide adducts of active hydrogen compounds.

More particularly, this invention relates to a process for such preparation employing a particular bimetallic compound as catalyst.

A large variety of products useful, for instance, as surfactants, solvents, and chemical intermediates, are prepared by the addition reaction (alkoxylation reaction) of alkylene oxides with organic compounds having one or more active hydrogen atoms. As an example, particular mention may be made of the alcohol ethoxylates and alkyl-substituted phenol ethoxylates prepared by the reaction of ethylene oxide with aliphatic alcohols or substituted phenols of about 8 to 20 carbon atoms, which ethoxylates are common nonionic detergent components of commercial cleaning formulations for use in industry and in the home. An illustration of the preparation of such an aliphatic alcohol ethoxylate (represented by formula III below) by addition of a number (p) of ethylene oxide molecules (formula II) to a single alcohol molecule (formula I) is presented by the equation

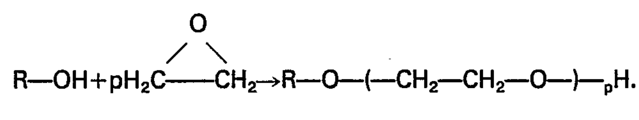

I II III

Alkylene oxide addition reactions are known to be promoted by contact with a catalyst, conventionally a catalyst of either acidic or basic character.

The present invention relates to an alkoxylation reaction catalyzed by certain bimetallic oxo compounds. Such substances have been known from the publication of Ph. Teyssie et al entitled "Catalysis with Soluble M—O—M'—O—M Bimetallic Oxides (*Chemtech.,* March 1977, p. 193). The bimetallic oxo compounds have not, however, been recognized as useful in promoting alkoxylation reactions.

The invention involves the discovery of a process for the production of alkylene oxide adducts (alkoxylates) characterized by a narrow alkylene oxide adduct distribution. Alkylene oxide addition reactions are known to produce a product mixture of various alkoxylate molecules having a variety of alkylene oxide adducts, (oxyalkylene adducts), e.g., having different values for the adduct number p in formula III above. The adduct number is a factor which in many respects controls the properties of the alkoxylate molecule, and substantial effort is often devoted to tailoring the adduct number distribution of a given product mixture to its intended service. In certain preferred aspects, the present invention is a process characterized by enhanced selectivity for the preparation of alkoxylate mixtures in which a relatively large proportion of the alkoxylate molecules have a number (p) of alkylene oxide adducts that is within a relatively narrow range of values. The alkoxylate products having such a narrow range distribution are preferred for use in detergent formulations. Narrow-range alkoxylate are also particularly valuable as chemical intermediates in the synthesis of certain carboxyalkylated alkyl polyethers and of certain alkyl ether sulphates.

Attempts made in the prior art to produce alkoxylates having a more narrow-range distribution of alkylene oxide adducts have centered upon processes for the preparation of alcohol alkoxylates, and most particularly upon the preparation of ethylene oxide adducts of higher ($C_8$ to $C_{20}$) aliphatic primary alcohols. The common conventional basic catalysts, i.e., compounds of the alkali metals, are known to be responsible for the production of alcohol ethoxylates having a relatively broad distribution. Conventional acid-catalyzed alkoxylation catalysts have long been recognized to produce alcohol ethoxylate products having a narrow distribution of alkylene oxide adducts. However, acid catalysis is known to have substantial disadvantage in several respects. For instance, the acids are often unstable, with limited life and effectivenesss as catalysts in the ethoxylation mixture. Both the catalysts themselves and their decomposition products catalyze side reactions producing relatively large amounts of polyethylene glycols, and also react directly with the components of the alkoxylation mixture to yield organic derivatives of the acids. Overall use of acid ethoxylation catalysts is known to result in relatively poor quality products.

A great deal of attention has recently been given in the art to processes which utilize basic compounds of the alkaline earth metals as catalysts for the preparation of alcohol alkoxylate products having a relatively narrow-range distribution. For instance, it has recently been reported (U.S. Patent Nos.4,210,764, 4,223,164, 4,239,917, 4,453,022, 4,453,023, 4,302,613 and 4,375,564 and the published European Patent Application Nos. 0026544, 0026546, 0026547, 0085167 and 0092256) that alkoxylation promoted by basic barium, strontium, calcium and magnesium compounds, either alone or with specified co-catalysts, yields an alkoxylate having a distribution which is more narrow or peaked than that of the product of an alkoxylation promoted by basic compounds of the Group I metals. Such products are still, however, considered to be less than optimal from the standpoint of overall product quality, requirements for catalyst removal and/or narrowness of product distribution.

It has now been found that certain bimetallic oxo compounds are useful as catalysts for the addition

reaction of alkylene oxides with alcohols and other organic compounds having one or more active hydrogen atoms.

Accordingly the invention relates to a process for the preparation of alkylene oxide adducts of active hydrogen containing compounds, which comprises reacting an alkylene oxide reactant with an active hydrogen reactant in the presence of a catalytically effective amount of a bimetallic oxo compound of the formula

$$(RO)_nM—O—M'—O—M(OR)_n$$

wherein each R is a hydrocarbyl group having from 1 to 30 carbon atoms, M' is a divalent metal selected from elements of Groups VIII, Ib, IIb, IIIb, and IVb of the Periodic Table, each M is a trivalent metal or a tetravalent metal, and n is 2 if the adjacent M is a trivalent metal or 3 if the adjacent M is a tetravalent metal.

It has further been found that the use of such bimetallic oxo compounds as alkoxylation catalysts provides a process for the preparation of an alkoxylate product, particularly an alkanol ethoxylate product, having an exceptionally narrow-range alkylene oxide adduct distribution. This product is of high quality (relatively free of by-products) and is characterized by a distribution which is notably more narrow or peaked than that of products of conventional alkoxylation reactions catalyzed by basic compounds of either the alkali metals or the alkaline earth metals.

The attached drawing represents, in its one figure, a representative plot of the distribution of alkylene oxide adducts in products prepared by reaction of ethylene oxide with $C_{12}$ and $C_{13}$ primary linear aliphatic alcohols in the presence of each of three different catalysts. The curve designated A represents the typical distribution of a product prepared using a conventional basic alkali metal alkoxylation catalyst, specifically KOH; curve B represents the typical distribution of a product prepared using a conventional basic alkaline earth metal alkoxylation catalyst, specifically barium hydroxide; and curve C represents the distribution of a product prepared under practice in accordance with the invention.

On the horizontal axis is given the oxyethylene adduct number ranging from 1 to 10. On the vertical axis is given the weight percentage of product with a certain number of oxyethylene units, in the total reaction product mixture.

The invention is generally applicable to processes utilizing any alkylene oxide (epoxide) reactant containing one or more alkylene oxides having from two to about 20 carbon atoms. Specific examples of alkylene oxides suitable for use in the alkoxylation of active hydrogen containing compounds are well known in the art. Preference generally exists for the use of the lower, e.g. $C_2$ to $C_8$ alkylene oxides and for the use of the vicinal alkylene oxides. From the standpoint of commercial interest, specific mention may be made of the $C_2$ to $C_4$ vicinal alkylene oxides, including ethylene oxide, propylene oxide, and the 1,2- and 2,3-butylene oxides. Particularly preferred are ethylene oxide and propylene oxide, while use of ethylene oxide is considered most preferred. Mixtures of alkylene oxides are suitable, in which case the product of the invention will be a mixed alkoxylate.

Likewise, the active hydrogen reactants suitably utilized in the process of the invention include those known in the art for reaction with alkylene oxides and conversion to alkoxylate products. The suitable classes of active hydrogen reactants include alcohols, phenols, thiols, (mercaptans), amines, polyols, carboxylic acids, and mixtures thereof.

Among the suitable carboxylic acids, particular mention may be made of the mono- and dicarboxylic acids, both aliphatic (saturated and unsaturated) and aromatic. Specific examples include acetic acid, propionic acid, butyric acid, valeric acid, caproic acid, lauric acid, myristic acid, palmitic acid, staric acid, oleic acid, rosin acids, tall oil acids, terephthalic acid, benzoic acid, phenylacetic acid, toluic acid, acrylic acid, methacrylic acid, crotonic acid, maleic acid, and the like.

Among the suitable amines, particular mention may be made of primary, secondary and tertiary alkylamines and of alkylamines containing both amino and hydroxyl groups, e.g., N,N - di(n - butyl) - ethanolamine and tripropanolamine.

Among the suitable thiols, particular mention may be made of primary, secondary and tertiary alkane thiols having from 1 to about 30 carbon atoms, particularly those having from about 8 to 20 carbon atoms. Specific examples of suitable tertiary thiols are those having a highly branched carbon chain which are derived via hydrosulphurization of the products of the oligomerization of lower olefins, particularly those dimers, trimers, and tetramers and pentamers of propylene and the butylenes. Secondary thiols are exemplified by the lower alkane thiols, such as 2 - propanethiol, 2 - butanethiol, and 3 - pentanethiols, as well as by the products of the hydrosulphurization of the substantially linear oligomers of ethylene as are produced by the Oxo processes. Representative examples of thiols derived from ethylene oligomers include the linear carbon chain products, such as 2 - decanethiol, 3 - decanethiol, 4 - decanethiol, 5 - decanethiol, 3 - dodecanethiol, 5 - dodecanethiol, 2 - hexadecanethiol, 5 - hexadecanethiol, and 8 - octadecanethiol, and the branched carbon chain products, such as 2 - methyl - 4 - tridecanethiol. Primary thiols are typically prepared from terminal olefins by hydrosulphurization under free-radical conditions and include, for example, 1 - butanethiol, 1 - hexanethiol, 1 - dodecanethiol, 1 - tetradecanethiol and 2 - methyl - 1 - tridecanethiol.

Among the polyols, particular mention may be made of those having from 2 to about six hydroxyl groups. Specific examples include the alkylene glycols such as ethylene glycol, propylene glycol, hyxelene

glycol, and decylene glycol, the polyalkylene glycol ethers, such as diethylene glycol, triethylene glycol, propylene glycol, dipropylene glycol, and tripropylene glycol, glycerine, sorbitol, and the like.

The alcohols and phenols are today the principal reactants in commercial alkoxylate production and are preferred classes of active hydrogen reactants for purposes of the invention. Among the phenols, particular mention may be made of phenol and of alkyl-substituted phenols wherein each alkyl substituent has from one to 30 (preferably from one to 20) carbon atoms, for example, p - methylphenol, p - ethylphenol, p - hexylphenol, p - decylphenol, didecyl phenol and the like.

Acyclic aliphatic alcohols form a most preferred class of reactants. In this regard, it is found that the aliphatic alcohols benefit to a relatively great degree from the capabilities of the invention for the preparation of alkoxylates having narrow-range or peaked alkylene oxide adduct distributions. This is particularly true for the primary mono-hydric aliphatic alcohols, although secondary and tertiary alcohols as well as polyhydric alcohols are also very suitably utilized in the process of the invention. Preference can also be expressed, for reason of both process performance and commercial value of the product, for aliphatic alcohols having from one to 30 carbon atoms, with $C_6$ to $C_{24}$ alcohols considered more preferred and $C_8$ to $C_{20}$ alcohols considered most preferred As a general rule, the aliphatic alcohols may be of branched or straight chain structure, although preference further exists for alcohol reactants in which greater than 50 per cent, more preferably greater than 60 per cent and most preferably greater than 70 per cent of the molecules are of linear (straight-chain) carbon structure.

Specific examples of primary straight-chain monohydric aliphatic alcohols include ethanol, hexanol, octanol, dodecanol, pentadecanol, octadecanol and eicosanol. Specific examples of branched chain or secondary alcohols include isopropanol, isoheptanol, 3 - heptanol, isodecanol, 2 - methyl - 1 - nonanol, 2 - methyl - 1 - undecanol, 4 - tetradecanol, and 4 - hexadecanol.

Mixtures of active hydrogen reactants in general and mixtures of alcohols in particular are suitable for purposes of the invention and are often preferred for reasons of their commercial availability. Mixtures of higher (e.g., $C_8$ to $C_{20}$) monohydric acyclic aliphatic alcohols are known to be commercially prepared, for instance, by hydroformylation of olefins or by reduction of naturally occurring fatty esters. Specific examples of commercially available alkanol mixtures in the $C_9$ to $C_{18}$ range are the Neodol detergent alcohols, trademark of and manufactured by Shell Chemical Company, e.g., the products identified as Neodol 91 alcohols (predominantly in the $C_9$ to $C_{11}$ range), Neodol 23 alcohols, (predominantly $C_{12}$ and $C_{13}$ alcohols), Neodol 25 alcohols (predominantly $C_{12}$ to $C_{15}$), and Neodol 45 alcohols (predominantly $C_{14}$ and $C_{15}$).

For purposes of the invention, the alkylene oxides reactant and the active hydrogen reactant are necessarily contacted in the presence of the specified bimetallic oxo catalyst. Such materials are known to the art and are conventionally represented by the formula

$$(RO)_nM{-}O{-}M'{-}O{-}M(OR)_n$$

M in the above formula represents (individually in each occurrence) either a trivalent metal or atetravalent metal. Preferably, each M is independently selected from the group consisting of aluminium, titanium, boron, vanadium, scandium, germanium, yttrium, zirconium, tin, lanthanum and other members of the lanthanide series, hafnium, tantalum, tungsten, palladium, and antimony. More preferably, each M is selected from the group consisting of aluminium, titanium, boron and vanadium, and most preferably each M is either aluminium or titanium.

M' in the above formula represents a divalent metal selected from those divalent metals of the Groups VIII, Ib, IIb, IIIb and IVb of the Periodic Table (i.e, the elements of atomic number 26—32, 44—50, and 76—82). M' is preferably selected from the group consisting of cobalt, nickel, copper, zinc, rhodium, palladium, silver, cadmium, indium, tin, iridium, and platinum, and is more preferably selected from the group consisting of zinc, nickel, and cobalt. Most preferred as the M' metal are zinc and cobalt.

The R substituents in the above formula individually and independently represent a hydrocarbyl group and preferably an alkyl group. The carbon numbers of the R substituents have from one to 30 carbon atoms, particularly from one to 20 carbon atoms. Catalysts with lower alkyl (e.g., $C_1$ to $C_6$) R groups are most easily prepared and are very suitable for use in the invention. The use of catalysts having R substituents with carbon numbers in these ranges facilitates a homogeneous reaction mixture in which the catalyst is soluble in the active hydrogen containing reactant. It is not necessary, however, that the reaction involve homogeneous catalysis for purposes of the invention. In this regard one or more of the R substituents may suitably be of higher carbon number and/or the catalyst may be supported on a solid carrier, for example, silica, alumina, or a silica/alumina mixture, to produce a heterogeneous catalyst.

The subscripts n in the formula designate the number of OR groups bound to each M. If the adjacent M atom is trivalent n is 2, and if the adjacent M atom is tetravalent n is 3. It is to be emphasized that in any one bimetallic oxo molecule the two M atoms may be different, for example, one a trivalent metal and the other a different trivalent metal or a tetravalent metal. Likewise, the several R substituents in any one molecule may be the same or different organic radicals.

Methods for the preparation of bimetallic oxo compounds suitable for use in the invention are described in U.S. Patent No. 3,432,445, U.S. Patent No. 3,607,785, U.S. Patent No. 4,281,087, and publication Chemtech, March 1977, p. 193. A very convenient method for preparation of such a compound

in which R represents an alkyl radical involves the reaction of two mols of trivalent and/or tetravalent metal (M) alkoxide with one mol of the acetate of a divalent metal (M'), for instance, at elevated temperature (e.g., 200°C) and in the presence of a solvent (e.g., tetralin):

$$2(RO)_{n+1}M+M'(OAc)_2 \rightarrow (RO)_nM—O—M'—O—M(OR)_n+2ROOAC$$

If desired, the bimetallic oxo compounds may be prepared with one set of R groups and then one or more different R group(s) substituted into the molecule by alcoholysis reaction.

The bimetallic oxo compound is present in the reaction mixture in a catalytically effective amount, typically at least about 0.01%w (per cent by weight), based on the active hydrogen reactant. Although catalyst quantity is not narrowly critical, preference may be expressed for use of the catalyst in amount of at least about 0.1%w, while an amount between about 0.2 and 1.0%w is considered most preferred. Substantially greater quantities of bimetallic oxo catalyst, e.g., 10 or 20% w, are very suitable and may be preferred for applications involving heterogeneous catalysts.

In terms of processing procedures, the alkoxylation reaction in the invention may be conducted in a conventional manner. The active hydrogen reactant and the bimetallic oxo compound are very conveniently introduced into a reactor, followed by addition of that quantity of the alkylene oxide reactant necessary to produce an alkoxylate product of the desired mean or average adduct number, e.g., typically from less than one to about 30. In general terms, suitable and preferred process temperatures and pressures for reactions utilizing the bimetallic oxo catalysts are the same as in conventional alkoxylation reactions, between the same reactants, employing conventional catalysts. A temperature of at least about 60°C, particularly at least about 100°C, is typically necessary for a significant rate of reaction, while a temperature less than about 250°C, particularly less than about 200°C and most particularly less than about 170°C, is typically to minimize degradation of the product. Superatmospheric pressures are preferred for processes involving the lower (particularly $C_2$ to $C_4$) alkylene oxide reactants. While these procedures describe a batch mode of operation, the invention is equally applicable to a continuous process.

When the preferred $C_6$ to $C_{24}$ alkanols or the preferred alkyl-substituted phenols and the preferred $C_2$ to $C_4$ vicinal alkylene oxides are applied as reactants in the process of the invention, the alkoxylation reaction is preferably carried out at a temperature in the range from 130° to 200°C, while a temperature between 150° and 190°C is still more preferred. Considered most preferred is a reaction temperature in the range from about 165° to 175°C. A total pressure in the range from 0,7 to 10 barg is usually preferred for the reaction between such higher alkanols or substituted phenols and lower alkylene oxides. The alkanol or phenol reactant is generally a liquid and the alkylene oxide reactant is generally a vapour for such reactions. Alkoxylation is then suitably conducted by introducing gaseous alkylene oxide into a pressure reactor containing the liquid alkanol. Catalyst is very conveniently in solution in, or otherwise mixed with, the alkanol. For considerations of process safety, the partial pressure of the lower alkylene oxide reactant is preferably limited, for instance, to less than about 4 bar, and/or the reactant is preferably diluted with an inert gas such as nitrogen, for instance, to a vapour phase concentration of about 50 per cent or less. The reaction can, however be safely accomplished at greater alkylene oxide concentration, greater total pressure and greater partial pressure of alkylene oxide if suitable precautions, known to the art, are taken to manage the risks of explosion. A total pressure of between about 3 and 8 barg, with an alkylene oxide partial pressure between about 1 and 4 barg, is particularly preferred, while a total pressure of between about 3.5 and 6.5 barg, with an alkylene oxide partial pressure between about 1.5 and 3.5 barg, is considered more preferred.

The alkoxylate prepared in the process of the invention is typically a product of very acceptable quality, having a relatively low content of polyalkylene glycols and other by-products. Unlike the products of typical acid or base catalyzed reactions of the prior art, the product of the invention is of essentially neutral pH. The bimetallic oxo compounds do not impart significant acidic or basic character to the reactants or to the product. Accordingly, it is not necessary, as in conventional practice, to neutralize the alkoxylate product by addition of base or acid. In this regard, the neutral pH of the process is considered to be a further desirably feature of the invention from the standpoint of product quality.

The following Examples and Comparative Experiments are provided.

Example 1

An alkoxylation process in accordance with the invention was carried out using a bimetallic (zinc and aluminium) oxo catalyst. The catalyst was prepared by dissolving a mixture of 2 grams (0.011 mols) of anhydrous zinc acetate and 4.45 grams (0.022 mols) of aluminium isopropoxide in 100 ml tetralin, and heating the resulting solution at 200°C for 18 hours. After cooling to 25°C, the solvent was removed by evaporation under vacuum to produce 4.3 grams of a beige powder.

The active-hydrogen reactant for the alkoxylation was a Neodol 23 Alcohol (trademark of and sold by Shell Chemical Company), characterized as a mixture of primary, 80% linear (20% branched), aliphatic alcohols containing twelve and thirteen carbon atoms (about 45% $C_{12}$, 55% $C_{13}$). Initially, the liquid alcohol reactant was dried by heating under a nitrogen sparge at 130°C for one hour. Then, one gram (0.0026 mols) of the zinc and aluminium bimetallic oxo catalyst prepared as described above was added to the alcohol at

130°C and the mixture was maintained at that temperature and under nitrogen sparge for an additional hour.

The catalyst and alcohol mixture was transferred to an autoclave reactor which was then sealed, heated to 170°C and pressured to 5 barg with a mixture of ethylene oxide in nitrogen (40% ethylene oxide by mol). The reaction commenced without an induction period. Temperature in the autoclave was maintained at 170°C and ethylene oxide was added to the reactor system, upon demand, to maintain the 5 barg pressure. About 45 grams of ethylene oxide reacted over a three hour period. The reactor was then maintained at 170°C for an additional 30 minutes without further addition of ethylene oxide, to consume unreacted ethylene oxide in the system. The product was analyzed by combined GC/LC and found to have an average ethylene oxide adduct number (mols ethylene oxide reacted, divided by total mols of alcohol) of 1.5 and to contain 1.3%w polyethylene glycols (PEG). The distribution of the various adducts in the alkoxylate product of this Example is presented in the Table below and illustrated by Curve C in the attached drawing.

Example 2

The procedures of Example 1 were repeated for the preparation of a product having an average ethylene oxide adduct number of 3.0. The product contained 1.2%w PEG.

Comparative Experiments

Two comparative experiments illustrate that results of the use of a bimetallic catalyst according to the invention are distinguishable from results of the independent use as catalyst of compounds of the individual metals from which the bimetallic combination had been derived.

For one comparative experiment, 66 grams (0.340 mol) of Neodol 23 Alcohol were dried at 130°C for one hour under nitrogen sparge. One gram (0.0049 mol) of aluminium isopropoxide was added to the alcohol and the mixture then heated at 130°C for one additional hour under nitrogen sparge. The catalyst and alcohol mixture was transferred to an autoclave, heated to 170°C and pressurized to 5 barg with the ethylene oxide and nitrogen mixture. Temperature was maintained at 170°C and ethylene oxide was added upon demand. After 5 hours, only 12 grams of ethyleneoxide had been consumed, resulting in a product mixture having an average ethylene oxide adduct number of only 0.73 and containing 0.3%w PEG.

For the second comparative experiment, the same procedures were carried out utilizing 50 grams (0.258 mol) of alcohol and one gram (0.0055 mol) of zinc acetate. After 4 hours reaction in the autoclave, only 2 grams of ethylene oxide had reacted. The product mixture contained too little alkoxylate for proper analysis.

Example 3

An alkoxylation process in accordance with the invention was carried out using a bimetallic (cobalt and aluminium) oxo alkoxide catalyst. The catalyst was prepared by reacting cobalt acetate with aluminium isopropoxide (in molar ratio of 2:1) under the procedures for catalyst preparation described in Example 1.

One gram (0.0026 mol) of the catalyst was mixed with 50 grams (0.258 mol) of Neodol 23 Alcohol which had been dried at 130°C for one hour under a nitrogen sparge, and the mixture was heated at 140°C for one additional hour.

The catalyst and alcohol mixture was then transferred to the autoclave reactor, which was heated to 170°C and pressured to 5 barg with the mixture of ethylene oxide (40% mol) in nitrogen. Alkoxylation commenced and ethylene oxide was added upon demand. After four hours, 30.8 grams of ethylene oxide had been added, producing an ethoxylate product mixture having an average adduct number of 2.8.

Comparative Experiments

Experiments were also conducted under comparable procedures and conditions, but utilizing conventional catalysts and thus not in accordance with the invention. In one experiment A, a potassium hydroxide catalyst was used to prepare an ethoxylate of the Neodol 23 Alcohol reactant, having an average ethylene oxide adduct number of about 1.9. The adduct distribution is presented in the following Table and also indicated by curve A in the attached drawing.

In another experiment B, a barium hydroxide catalyst and the same reactants were used to prepare a product having an average adduct number of about 1.7. The distribution is shown in the Table and by curve B of the drawing.

Example 4

An alkoxylation process in accordance with the invention was practised using a bimetallic (zinc and aluminium) catalyst supported on a silica carrier. The carrier was a Davison 57 silica (30—100 mesh) which had been dried at 300°C under full vacuum for 18 hours. For preparation of the supported catalyst, one gram of a zinc/aluminium bimetallic oxo compound, prepared as described in Example 1, was dissolved in 50 grams of dry xylene and the solution added to 10 grams of the silica. The mixture was slurried at 85°C for one hour. The solids were filtered, washed several times with xylene and dried under vacuum at 80°C for one hour, to obtain a pale yellow powder.

For alkoxylation, three grams of the supported catalyst powder were added to 50 grams of

active-hydrogen reactant (Neodol 23 Alcohol). The process was carried out using conditions and procedures described in Example 1. A total of twenty-five grams of ethylene oxide reacted with the alcohol over a four-hour period to produce an ethoxylate mixture having an average adduct number of 1.5. Distribution of the several adducts was essentially the same as that of the product of Example 1.

TABLE

Catalyst

| Adduct number p | | $Zn^{+2}/Al^{+3}$ bimetallic oxo catalyst | Comparative experiments | |
|---|---|---|---|---|
| | | | KOH | $Ba(OH)_2$ |
| 0 | (unreacted alcohol) | 21.9%w | 28.9%w | 29.5%w |
| 1 | | 24.9 | 13.1 | 12.9 |
| 2 | | 24.5 | 13.2 | 15.8 |
| 3 | | 15.8 | 11.5 | 15.8 |
| 4 | | 7.3 | 8.9 | 12.3 |
| 5 | | 1.9 | 6.5 | 7.3 |
| 6 | | 1.6 | 4.8 | 3.4 |
| 7 | | 0.9 | 3.5 | 1.8 |
| 8 | | 0.6 | 2.6 | 0.7 |
| 9 | | 0.4 | 2.0 | 0.3 |
| 10 | | 0.3 | 1.4 | 0.1 |
| 11 | | 0.1 | 1.1 | |
| 12 | | | 0.7 | |
| 13 | | | 0.6 | |
| 14 | | | 0.4 | |
| 15 | | | 0.3 | |

**Claims**

1. A process for the preparation of alkylene oxide adducts of active hydrogen containing compounds which comprises reacting an alkylene oxide reactant with an active hydrogen reactant in the presence of a catalytically effective amount of a bimetallic oxo compound of the formula

$$(RO)_nM—O—M'—O—M(OR)_n,$$

wherein each R is a hydrocarbyl group having from 1 to 30 carbon atoms, M' is a divalent metal selected from the group consisting of elements of Groups VIII, Ib, IIb, IIIb, and IVb of the Periodic Table, each M is a trivalent metal or a tetravalent metal, and each n is 2 if the adjacent M is trivalent or 3 if the adjacent M is tetravalent.

2. A process as claimed in Claim 1, wherein M is trivalent or tetravalent metal selected from the group consisting of aluminium, titanium, boron, vanadium, scandium, germanium, yttrium, zirconium, tin, lanthanum and other members of the lanthanide series, hafnium, tantalum, tungsten, palladium, and antimony.

3. A process as claimed in Claim 1 or 2, wherein each M' is a divalent metal selected from the group consisting of cobalt, nickel, copper, zinc, rhodium, palladium, silver, cadmium, indium, tin, iridium, and platinum.

4. A process as claimed in Claims 1—3, wherein each R is a $C_1$ to $C_{30}$ alkyl group.

5. A process as claimed in Claim 4, wherein R is a $C_1$ to $C_6$ alkyl group.

6. A process as claimed in Claims 1—5, wherein the alkylene oxide reactant comprises one or more $C_2$ to $C_8$ vicinal alkylene oxides.

7. A process as claimed in Claim 6, wherein the alkylene oxide reactant comprises one or more $C_2$—$C_4$ vicinal alkylene oxides.

8. A process as claimed in Claims 1—7, wherein the active hydrogen reactant comprises one or more alcohols, phenols, and thiols.

9. A process as claimed in Claim 8, wherein the active hydrogen reactant is selected from the class consisting of acyclic aliphatic alcohols having from one to 30 carbon atoms, phenol, and alkyl-substituted phenols wherein each alkyl substituent has from one to 20 carbon atoms.

10. A process as claimed in Claim 9, wherein the alcohols are $C_6$ to $C_{24}$ alcohols.

11. A process as claimed in Claim 10, wherein the alcohols are $C_8$ to $C_{20}$ alcohols.

12. A process as claimed in Claim 11, wherein the alcohols are one or more $C_8$ to $C_{20}$ monohydric acyclic aliphatic primary alcohols.

**Patentansprüche**

1. Verfahren zur Herstellung von Alkylenoxid-Addukten von aktive Wasserstoffatome enthaltenden Verbindungen durch Umsetzung eines Alkylenoxids mit einem aktive Wasserstoffatome enthaltende Reaktionspartner in Gegenwart einer katalytische wirksamen Menge einer Oxoverbindung mit zwei Metallen der Formel:

$$(RO)_nM—O—M'—O—M(OR)_n,$$

worin R eine Kohlenwasserstoffgruppe mit 1 bis 30 Kohlenstoffatomen ist, M' ein zweiwertiges Metall aus der Gruppe VIII, Ib, IIb, IIIb, und IVb des Periodensystems ist und M jeweils ein drei- oder vierwertiges Metall bedeutet, wobei im Falle von M dreiwertiges Metall n 2 ist und im Falle von M vierwertiges Metall n 3 ist.

2. Verfahren nach Anspruch 1, worin M ein drei- bzw. vierwertiges Metall in Form von Aluminium, Titan, Bor, Vanadium, Scandium, Germanium, Yttrium, Zirkonium, Zinn, Lanthan, Lanthaniden, Hafnium, Tantal, Wolfram, Palladium oder Antimon ist.

3. Verfahren nach Anspruch 1 oder 2, worin M' ein zweiwertiges Metall in Form von Cobalt, Nickel, Kupfer, Zink, Rhodium, Palladium, Siber, Cadmium, Indium, Zinn, Iridium oder Platin ist.

4. Verfahren nach Anspruch 1 bis 3, worin R eine $C_1$- bis $C_{30}$-Alkylgruppe ist.

5. Verfahren nach Anspruch 4, worin R eine $C_1$- bis $C_6$-Alkylgruppe ist.

6. Verfahren nach Anspruch 1 bis 5, worin das Alkylenoxid ein oder mehrere $C_2$ bis $C_8$ vicinale(s) Alkylenoxid(e) umfaßt.

7. Verfahren nach Anspruch 6, worin das Alkenoxid ein oder merhere $C_2$ bis $C_8$ vicinale(s) Alkenoxid(e) umfaßt.

8. Verfahren nach Anspruch 1 bis 7, worin die ein aktives Wasserstoffatom enthaltende Verbindung ein oder mehrere Alkohol(e), Phenol(e) oder Thiol(e) sein kann.

9. Verfahren nach Anspruch 8, worin der aktives Wasserstoffatom enthaltende Reaktionspartner ausgewählt sein kann aus acyclischen aliphatischen Alkoholen mit 1 bis 30 Kohlenstoffatomem, Phenol und alkylsubstituierten Phenolen, wobei jeder Alkylsubstituent 1 bis 20 Kohlenstoffatome enthält.

10. Verfahren nach Anspruch 9, worin die Alkohole $C_6$- bis $C_{24}$-Alkohole sind.

11. Verfahren nach Anspruch 10, worin die Alkohole $C_8$- bis $C_{20}$-Alkohole sind.

12. Verfahren nach Anspruch 11, worin die Alkohole ein oder mehrere $C_8$ bis $C_{20}$ einwertige(r) acyclische(r), aliphatische(r), primäre(r) Alkohol(e) sind.

**Revendications**

1. Procédé de préparation de produits d'addition d'oxyde d'alcoylène de composés contenant de l'hydrogène actif dans lequel on fait réagir un réactif oxyde d'alcoylène avec un réactif à hydrogène actif en présence d'une quantité catalytiquement efficace d'un composé oxo bimétallique de formule

$$[(RO)_nM—O—M'—O—M(OR)_n]$$

où chaque R est un groupe hydrocarbyle ayant de 1 à 30 atomes de carbone, M' est un métal bivalent choisi dans le groupe constitué par les éléments des groupes VIII, Ib, IIb, IIIb, et IVb de la Classification Périodique, chaque M est un métal trivalent ou un métal tétravalent, et chaque n vaut 2 si le M adjacent est trivalent ou 3 si le M adjacent est tétravalent.

2. Procédé selon la revendication 1, où M est un métal trivalent ou tétravalent choisi parmi le groupe constitué par l'aluminium, le titane, le bore, le vanadium, le scandium, le germanium, l'yttrium, le

zirconium, l'étain, le lanthane et les autres membres de la série des lanthanides, l'hafnium, le tantale, le tungstène, le palladium et l'antimoine.

3. Procédé selon la revendication 1 ou 2, dans lequel chaque M' est un métal bivalent choisi dans le groupe constitué par le cobalt, le nickel, le cuivre, le zinc, le rhodium, le palladium, l'argent, le cadmium, l'indium, l'étain, l'iridium et le platine.

4. Procédé selon les revendications 1—3, dans lequel chaque R est une groupe alcoyle en $C_{1-30}$.

5. Procédé selon la revendication 4, dans lequel R est un groupe alcoyle en $C_{1-6}$.

6. Procédé selon les revendications 1—5, dans lequel le réactif oxyde d'alcoylène comprend 1 ou plusieurs oxydes d'alcoylène vicinaux en $C_{2-8}$.

7. Procédé selon la revendication 6, dans lequel le réactif oxyde d'alcoylène comprend un ou plusieurs oxydes d'alcoylène vicinaux en $C_{2-4}$.

8. Procédé selon les revendications 1—7, dans lequel le réactif à hydrogène actif comprend un ou plusieurs alcools, phénols et thiols.

9. Procédé selon la revendication 8, dans lequel le réactif à hydrogène actif est choisi dans la classe constituée par les alcools aliphatiques acycliques ayant de 1 à 30 atomes de carbone, le phénol et les phénols alcoyle-substitués, où chaque substituant alcoyle a de 1 à 20 atomes de carbone.

10. Procédé selon la revendication 9, dans lequel les alcools sont des alcools en $C_{6-24}$.

11. Procédé selon la revendication 10, dans lequel les alcools sont des alcools en $C_{8-20}$.

12. Procédé selon la revendication 11, dans lequel les alcools sont un ou plusieurs alcools primaires aliphatiques acycliques monohydriques en $C_{8-20}$.

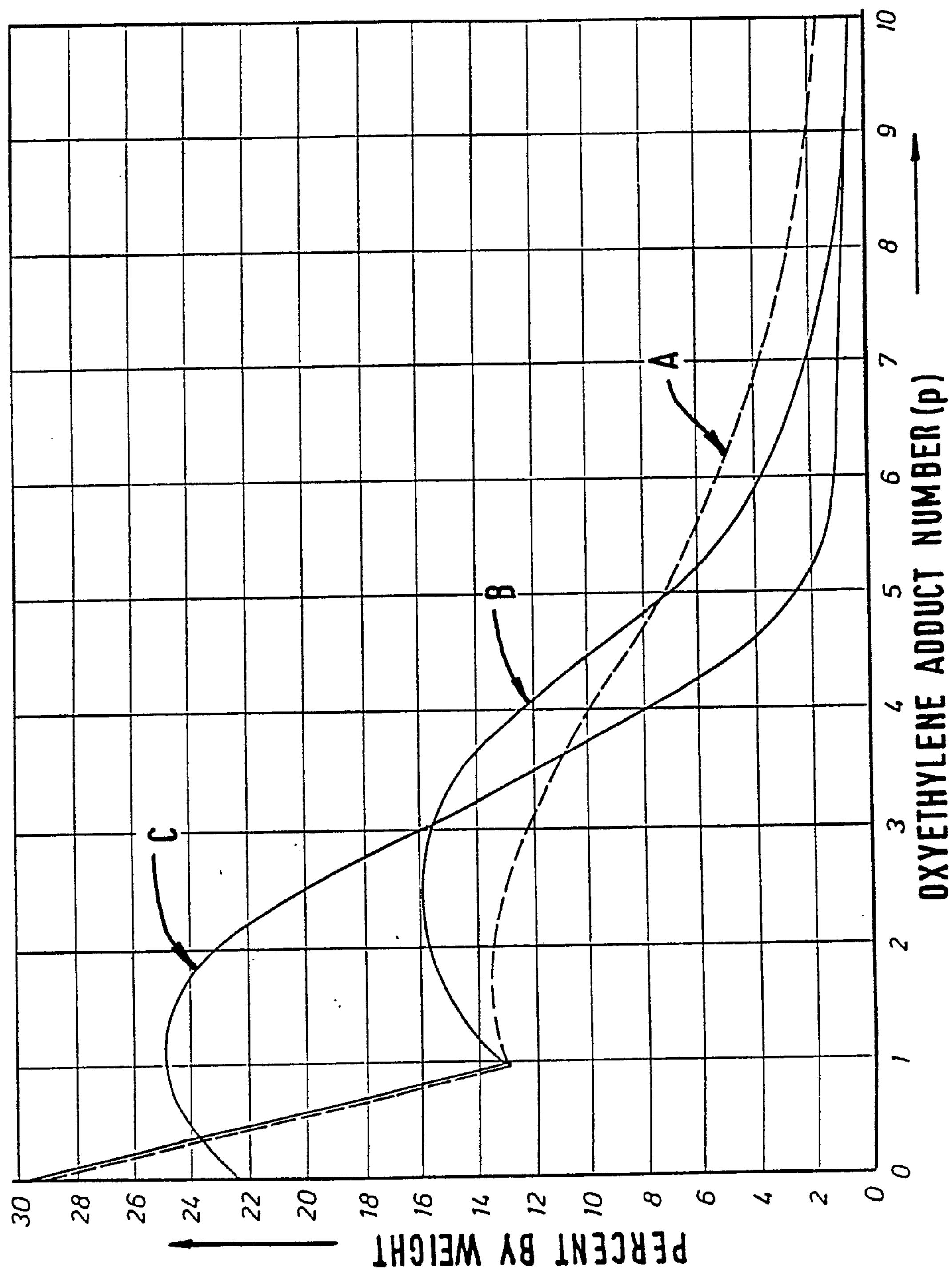
A
B
C
OXYETHYLENE ADDUCT NUMBER (p)
0
1
2
3
4
5
6
7
8
9
10
PERCENT BY WEIGHT
0
2
4
6
8
10
12
14
16
18
20
22
24
26
28
30